# EUROPEAN PATENT APPLICATION

(11) **EP 0 697 168 A1**
(43) Date of publication of application: **21.02.1996**
(21) Application number: 95810513.2
(22) Date of filing: 16.08.1995
(51) Int. Cl.: A01H 1/04, A01H 3/04, A01H 5/12

(54) **Low nitrate lettuce**

(30) Priority: 18.08.1994 GB 9416763
(71) Applicant: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Koedijk, Jeanette Martine, NL-2675 BH Honselersdijk (NL); Van Doorn, Johannes Elisabert, NL-1613 AL Grootebroeck (NL); Van Holst, Gerrit Jan, NL-1602 DN Enkhuizen (NL)

(57) **Abstract**

The present invention provides lettuce heads which contain less than or equal to 0.25% (w/w) nitrate at the time of harvest, the plants from which they are obtained having been grown and harvested under natural light at a day-length of not more than 10.5 hours. *Interalia*, the invention also provides the plants from which the heads are obtained and the seeds, and other propagating material, such as somatic embryos for example, capable of yielding such plants.

## Description

The invention relates to lettuce (*Lactuca sativa*) plants having a low nitrate content and in particular to those lettuces the heads of which have a low nitrate content when the plants from which they are obtained are grown and harvested in late autumn/winter months in countries having a latitude of about 50 to about 60°.

The nitrate content of lettuce heads substantially depends on the day-length. Lettuce grown at shorter day-length tends to have a relatively higher nitrate content. In areas of more northern latitude this may in winter-time give rise to lettuce heads having a relatively high nitrate content. The health authorities regard a high nitrate content as undesirable. In the European Union for example, the present maximum allowable nitrate content of lettuce for human consumption is to be reduced. This creates problems for lettuce growers in countries with short day-length (in winter-time, for example). It is therefore necessary to make available lettuce plants with heads having a low nitrate content.

Known lettuce cultivars such as *Grosse Brune Tetue, Reichenauer Winter, and Trocadero Light 76*, for example are intrinsically capable of yielding heads having a low nitrate content, but only under relatively high light intensities and durations, such as those not experienced in countries having a latitude of about 50 to about 60° during the months of October to about March. If such known plants are grown in such countries during this time period they do not form heads.

Whilst the skilled man does not need a description of a lettuce "head" in order to be able to recognize one, by lettuce "head" is meant a substantially spherical or uni-directionally elongated spheroidal edible mass consisting of relatively tightly packed overlapping leaves, the mass being substantially axially disposed within the lettuce. Accordingly, the term "head" excludes relatively loosely packed or substantially isolated peripheral leaves which are generally circumferentially located around the head. Typical expressions used to describe lettuces which have heads are crisp heart and butter heart. It is to be appreciated that certain lettuces may not form heads under conditions of low light intensity or very poor nutrient supply.

According to the present invention there is provided lettuce heads which contain less than or equal to 0.25% (w/w) nitrate at the time of harvest, the plants from which they are obtained having been grown and harvested under natural light at a day-length of not more than 10.5 hours.

By "day-length" is meant the interval between the centre of the sun being six degrees under the horizon. By "natural light" is meant non-artificial light, in particular electromagnetic radiations having a wavelength of from about 0.3 to about 1µm. In particular "natural light" means such electromagnetic radiations having a wavelength of from about 400 to about 800nm.

The nitrate content of the lettuce heads can easily be determined according to standard tests, one of which is described below.

*Inter alia*, the invention also provides the plants from which the heads are obtained and the seeds, and other propagating material, such as somatic embryos for example, capable of yielding such plants. The term "propagating material" as used herein comprises plant parts such as totipotent cells growing in culture, seeds, leaf, stem, roots, shoots and the like, protoplasts, somatic embryos, anthers, stamens, and petioles.

A typical day-length experienced by growing lettuces according to the invention is between about 7.5 and 10.5 hours, with the quality of the light being of relatively low intensity, typically less than an average of about 7,00J/cm⁻per day (averaged over 10 days). It is very surprising that lettuces having such a low nitrate concentration as those presently claimed are able to form heads under conditions of such low light intensity and duration.

It is preferred that the lettuce heads of the invention weigh at least 60 grams at the time of their harvest. Typically such heads will weigh between 100 and 300 grams.

The invention still further provides lettuce plants the seedlings of which have dark green cotyls when grown in the presence of about 10mM alkaline metal chlorate. By "dark green" is meant a colour having a reading of between 42 and 55 (corresponding to 10GY to 5BG) judged according to the well known "Munsell hue".

The invention still further provides lettuce roots containing an NADH dependant nitrate reductase which, in the presence of non-limiting quantities of nitrate, is capable of catalyzing the formation of at least 3.7µmol nitrite per gram fresh weight of root per hour and/or lettuce heads containing an NADPH dependant nitrate reductase which, in the presence of non-limiting quantities of nitrate, is capable of catalyzing the formation of at least 6.1µmol nitrite per gram fresh weight of head per hour. It is preferred that such roots and heads have been obtained from plants which have been grown and harvested under natural light at a day-length of not more than 10.5 hours. The determinations of NADPH and NADH dependant nitrate reductase activities are well known to the skilled man (see below). Accordingly, the present invention still further provides plants each of which comprises such roots in conjunction with such a head. Such lettuce plants preferably contain heads which contain less than or equal to 0.25% (w/w) nitrate at the time of harvest, the plants having been grown and harvested under natural light at a day-length of not more than 10.5 hours.

The invention still further provides a method of selecting low nitrate lettuces comprising germinating lettuce seeds or growing the resulting seedlings in the presence of about 10mM alkaline metal chlorate and selecting those seedlings which have non necrotized - substantially shrunken cotyledons, and substantially unextended hypocotyls. Particularly suitable chlorates for use in such a method include potassium and sodium chlorates, with the potassium salt being the most preferred. Excluding the cultivar WN22, the said method enables selection of lettuce plants which contain less than or equal to 0.25% (w/w) nitrate at the time of harvest, the plants having been grown and harvested under natural light at a day-length of not more than 10.5 hours.

Lettuce plants of the invention can be obtained by:
(i) crossing
   (a) line N38 or the progeny of a cross of line N38 with a lettuce plant having the desired agronomical value, any F2 and subsequent generations of said progeny having been selected for low nitrate content;
      with
   (b) line WN22 or the progeny of a cross of line WN22 with a lettuce plant having the desired agronomical value, any F2 and subsequent generations of said progeny having been selected for low nitrate content;
(ii) harvesting the seeds of said cross, growing them and selecting the lettuce plants with heads having a low nitrate content;
(iii) optionally crossing selected plants from step (ii) with lettuce plants having the desired agronomical value, and selecting from the progeny of the latter cross plants having heads with a low nitrate content;
(iv) selfing the selected plants according to steps (ii) or (iii) repeatedly and selecting in each generation the plants having heads with a low nitrate content;
(v) whereby selfed plants obtained and selected according to step (iv) may be crossed with lettuce plants having the desired agronomical value, provided the progeny thereof is screened for nitrate content, and the plants of the progeny having a low nitrate content, are then selfed and selected according to step (iv) and such selfing process repeated till lettuce plants with heads of the desired nitrate content are obtained.

Whenever in steps (i)(a), (i)(b), (iii) and/or (v) the progeny of lettuce plants having an appropriate value is employed, such progeny will preferably have been selfed to the F3 or subsequent generations. The plants selected from the progeny for further breeding will preferably have the lowest nitrate content found in that generation.

Likewise, in steps (ii), (iii), (iv) or (v) the plants selected for further breeding will preferably have the lowest nitrate content found in that generation.

It is of course not essential to grow the lettuce plants according to the invention in seasons with relatively short day-length resp. to harvest the lettuce plants arising during the breeding program at days having a length of 10.5 hours or less. It is indeed possible to calculate the approximate nitrate content of lettuce heads to be harvested at day-lengths of 10.5 hours (or less) from the nitrate content found for lettuce heads harvested at longer day-lengths. Such conversion principles are known. It will however be appreciated that growing the lettuce under natural light and harvesting it at day-lengths of 10.5 hours or less will provide the most reliable information on nitrate content in the plants actually grown under such conditions.

The selfing according to step (iv) is preferably carried out to at least the F4 generation. In general it may be indicated to self to the F5 generation, or even - and preferably - to the F6 generation, to obtain the lettuce plants having heads with the desired nitrate concentration. Such selfing is also indicated to obtain lettuce having the desired uniformity.

The trait for low nitrate content provided by line N38 may be polygenic. The trait for low nitrate content provided by line WN22 may be monogenic. The latter has been obtained by somaclonal variation of a cultivated variety (Nanda) as indicated below.

It will be appreciated from the above that the invention provides a genetic complex comprising a trait for low nitrate content provided by line N38 and a trait for low nitrate content provided by line WN22 and the use of such a genetic complex in lettuce plants in the production of lettuce heads having a nitrate content of less than or equal to 0.25% (w/w) nitrate at the time of harvest, the plants having been grown and harvested under natural light at a day-length of not more than 10.5 hours. The traits may be provided by the nitrate reductase gene loci in each of the lines.

The genetic complex may be introduced in lettuce plants having the desired agronomical value in a manner known *per se*, conveniently by breeding and selecting according to the process disclosed hereinabove.

The nitrate content of the lettuce heads may also be dictated by factors other than the day-length at which they are grown and harvested, such as the mean global radiation (in particular over a period of ten days before harvest), the soil in which the lettuce plants are grown (e.g. normal soil, peat soil or hydroculture), the composition of the nutrient solution employed and the like. However, the desired nitrate concentration can be obtained when crossing, selfing and selecting the plants according to the process of the invention and bearing in mind that variation of the nitrate content may depend on such other factors. It may for example be indicated to grow the lettuce plants in several of the growth media normally contemplated, to secure that the desired low nitrate content is achieved, substantially irrespective of the grown medium employed.

The genetic complex obtained from lines N38 and WN22 and responsible for the low nitrate content is capable of ensuring that lettuce heads having the desired low nitrate content when grown and harvested under natural light at a day-length of 10.5 hours or less, for example, a day-length at harvest of from 7.5 hours to 10.5 hours, may be produced.

Preferably, where the low nitrate lettuces of the invention are obtained by breeding the WN22 and N38 progenitors indicated above, in each selection step those plants will be selected giving the lowest nitrate content which ensures that all elements of the said genetic complex are transferred to the next generation.

N38 and WN22 were deposited on August 10, 1994 with the National Collections of Industrial and Marine Bacteria Ltd., International Depository Authority, St Machar Drive, Aberdeen, Scotland, and received the designation numbers NCIMB 40676 and NCIMB 40677 respectively. Where permissible under the corresponding patent law regulations (Rule 28EPC, for example), the availability of these deposits to third parties is restricted to an expert intermediary either nominated by the president of the appropriate Patent Office or by the third party provided that the third party nomination is approved by the applicant.

The invention will be further apparent from the following specific description.

### Determination of nitrate content.

Lettuce heads weighing usually between 150 and 300 gram are put into blender and an equal weight of water is added. The mixture is thoroughly blended and the homogenate filtered through filter paper. The thus clarified samples are stored at -18° C prior to further analysis. After thawing and dilution, the nitrate concentration is analysed either by an HPLC method (Alltech Chromatography catalog 300, 1993, page 466, application 6151, Alltech Associates Inc., 2051 Wankegan Road, Deerfield, IL 60015, USA) or by a colorimetric method (A.S. Baker and R. Smith; J. Agr. Food Chem. 1969 Volume 17, page 1284 to 1287). The results are calculated as mg nitrate per kg fresh weight of lettuce material (= ppm).

### Preparation of low nitrate lettuce cultivars

Leaves of young plants with four leaves of the variety Nanda (a commercially available cultivated variety) were surface-sterilized, cut in 2 cm pieces and plated on a B-5 medium (O.L. Gamborg, D. Everleigh; Can. J. Biochem. (1968) 46; 417-421) with 0.7 % agar, 2 % sucrose, 1 mg/l naphthylene acetic acid and 0.5 mg/l 6-benzyladenine as described by R. Alcohero; Hort. Science (1983) 18: 305-307. After one month of growth at 25° C in the dark, a lot of white friable callus is formed. A portion of this callus is transferred into 100 ml Erlenmeyer flasks containing 20 ml B-5 medium with 2 % sucrose, 1 mg/l naphthylene acetic acid and 0.5 mg/l 6-benzyladenine.

The suspension cell cultures are sub-cultured ("passaged") at weekly intervals. After three weeks the cells from the suspension culture are placed on a solid B-5 medium containing 0.7 % (w/w) agar, 2 % (w/w) sucrose and 2 mg/l 6-benzyladenine.

Callus that turn green are monthly subcultured. After two months, small shoots are transferred to MS medium (T. Murashige, F. Skoog; Physiol. Plant (1962) 15: 473-497) containing 0.7 % (w/w) agar and 3 % (w7w) sucrose, in the absence of added hormones. Shoots that grow and develop roots are transferred to soil.

Regenerated (R₁) plants are allowed to flower and produce seed which is harvested. At least four seedlings (R₂) - at a four true leave stage - from each R₁ plant are tested for their nitrate content. Forty-six plants with a nitrate content of less than 80 % of that of Nanda are selected. These plants produce seeds. Twelve seedlings (R₃) from each selected R₂ plant are tested for their nitrate content at a two true leave stage. Four lines produce plants with a low nitrate content and of these lines twenty plants are grown to marketable head size in the early spring under glass-house cultivation conditions. Harvest is on April 11, 1989. The nitrate content of marketable heads is measured. Two lines of R₃ plants have a nitrate content significantly lower than that of Nanda. The line having the lowest nitrate content is selected and designated as WN22.

Mean nitrate content of Nanda (n=32): 3410 mg nitrate/kg, standard deviation 270 mg/kg (mean lettuce size 220g). Mean nitrate content of WN22: 2,590 mg/kg (n=20) standard deviation 240 mg/kg (mean lettuce size 211g).

WN22 is a homozygous R₃ line that has a nitrate content of 76 % of that of Nanda. Nanda has an average nitrate content of 91 % of that of Panvit (U. Behr. PhD thesis, Universität Hannover, BRD, 1988, p. 38).

### Preparation of very low nitrate lettuce cultivars

Line N38 is crossed with an experimental lettuce line having suitable agronomical value (designated Jimmy). The resulting plants are selfed to generation F3, and the nitrate content determined as indicated above. Of each generation, the plants having the lowest nitrate content, are selected. The selected plants of generation F3 are then crossed with line WN22, the thus obtained plants selfed and from the subsequent generations the lines having lowest nitrate content are each time selected. The selfing is repeated to the F6 generation.

The following results are obtained (growing was effected in the greenhouse located at 52° latitude and 4°15' longitude).
1. **Growth in soil**
day-length at harvest: 10 hours
lettuce (= head plus peripheral leaves) weight: at least 150 g

| | | | |
|---|---|---|---|
| sowing date: | 07.10.91 | 07.10.92 | 12.10.93 |
| harvest date: | 15.02.92 | 12.02.93 | 17.02.94 |

| **Line** | **ppm nitrate** | | |
|---|---|---|---|
| Nanda | 3630 | ----- | ----- |
| Kylie (standard commercial line) | ----- | 3960 | 3980 |
| N38 | 2860 | 2920 | 2990 |
| WN22 | 3260 | 3000 | 3030 |
| F4 (F3 N38*Jimmy)* WN22 | 2820 | ----- | ----- |
| F5 | ----- | 2700 | ----- |
| F6 | ----- | ----- | 2450 |

2. **Growth in hydroculture**
day-length at harvest: 10.5 hours
lettuce (= head plus peripheral leaves) weight: at least 150 g

| | | |
|---|---|---|
| sowing date: | 26.08.92 | 26.08.93 |
| harvest date: | 20.10.92 | 20.10.93 |

| **Line** | **ppm nitrate** | |
|---|---|---|
| Kylie | 3730 | 3930 |
| N38 | 2770 | 2530 |
| WN22 | ----- | 2640 |
| F5 (F3 N38*Jimmy)* WN22 | 2320 | ----- |
| F6 | ----- | 2040 |

### Determination of nitrate reductase activity in lettuce.

The nitrate content of lettuce is largely dictated by the rate of nitrate uptake by roots and the subsequent accumulation or metabolic conversion of such nitrate into organic nitrogen containing compounds. The conversion of nitrate into organic Nitrogen is catalyzed by the enzyme nitrate reductase, which exists in multiple forms in different plant compartments. Nitrate reductase is an allosteric enzyme which is regulated *inter* alia by light, hormones and amino acids.

Lettuces are separated into essentially two components: leaves and roots which are independently homogenized for two minutes in a HEPES based buffer at a ratio of 1 part lettuce material per 3 parts buffer (w/w). The HEPES based buffer contains HEPES buffer (pH 8.2); 0.1M HEPES, 1mM EDTA, 1mM Na₂MoO₄, 1mM PMSF (in 2 propanol), 2mM β-mercaptoethanol and 0.1g PVP-40 per gram fresh weight of lettuce material.

The homogenate is filtered, centrifuged (10,000g for 3 minutes) and left to stand on ice for 3 minutes before being used in the measurement of nitrate reductase activity. 20µl of lettuce material extract is added to 220µl of substrate which, in the case of the root derived material, is comprised by 0.1M KPO₄ buffer (pH 7.5), 10mM KNO₃, 200µM NADH and 0.1mM FAD. In the case of leaf derived material the NADH is replaced by NADPH. Nitrate reductase activity is determined by monitoring the change in NADH/NADPH absorbance at 340nm as a function of time.

### Selection of low-nitrate lettuce with potassium chlorate

The low nitrate lines N38, WN22, lower nitrate cultivars according to the present invention, and high nitrate cultivars are evaluated for differences in nitrate reductase activity during germination and early seedling development. Nitrate reductase activity of seedlings can indirectly be determined by screening seedling development and quality in the presence of potassium chlorate. Nitrate reductase is capable of converting chlorate into chlorite which is toxic. Seedlings with a relatively high nitrate reductase activity suffer relatively more from chlorite intoxication than do seedlings having a relatively low reductase activity, which may grow almost normally in the presence of chlorate.

The susceptibility of the various cultivars to chlorate intoxication is assessed as follows. Seeds from cultivars are germinated in transparent germination boxes (3 layers paper saturated with 35ml water or 0.5mM KClO₃) for two days under natural light conditions at 22° C. After two days all seeds are germinated and the seeds are fed at two-day intervals with a standard (N3) feeding solution including 7.5 mM nitrate +/-10 mM KClO₃.

Table 1 presents the seedling development and quality of various lettuce cultivars, varying maximally in nitrate content, on N3 substrate in the presence and absence of 10 mM KClO3.

From the table it is clear that high nitrate cultivars hardly respond to chlorate early during seedling development. The hypocotyledon length is slightly reduced but the cotyledon size and colour is comparable in the presence and absence of chlorate in the feeding solution. It is concluded that high-nitrate cultivars have a low nitrate-reductase activity in hypocotyledons and cotyledons. The low nitrate lines WN22 and N38 react heavily but differently to chlorate. WN22 and N38 hypocotyledons become respectively very and intermediately short in the presence of chlorate. On the other hand, the cotyledons of N38 grow as in the absence of chlorate while those of WN22 remain very small. Cotyledons of N38 are covered with yellow necrotic lesions while WN22 cotyledons remain dark-green.

The phenotype of N38 and WN22 seedlings in the presence of chlorate suggests that both lines have a high nitrate-reductase activity in separate plant parts. N38 seedlings appear to have a relatively high reductase activity in cotyledons in particular. WN22 seedlings appear to have a relatively high reductase activity in the non-green plant parts, such as stems and roots.

The Table also shows the effect of chlorate on the presently claimed cultivars, which as indicated above may be derived in a non obvious manner from N38 and WN22. From the table it appears that WN22 derived cultivars react to chlorate in a similar manner to the WN22 cultivar itself insofar as chlorate induces the production of relatively short seedlings having small dark-green cotyledons. The chlorate expressed WN22 phenotype appears to be dominant to the N38 phenotype, as the cultivars LS and line N, both WN22 x N38 recombinations, have the WN22 character. This "dominance" of WN22 over N38 is in agreement with our hypothesis that WN22 and N38 seedlings have a relatively high reductase activity in different plant regions, viz, stems and roots (WN22) and green tissues (N38).

The Table also indicates the effect of chlorate treatment on the nitrate reductase activity of three low nitrate reference cultivars, all of which behave similarly to N38 in this respect. i. e. all have normal light-green cotyledons with necrotic lesions in the presence of chlorate.

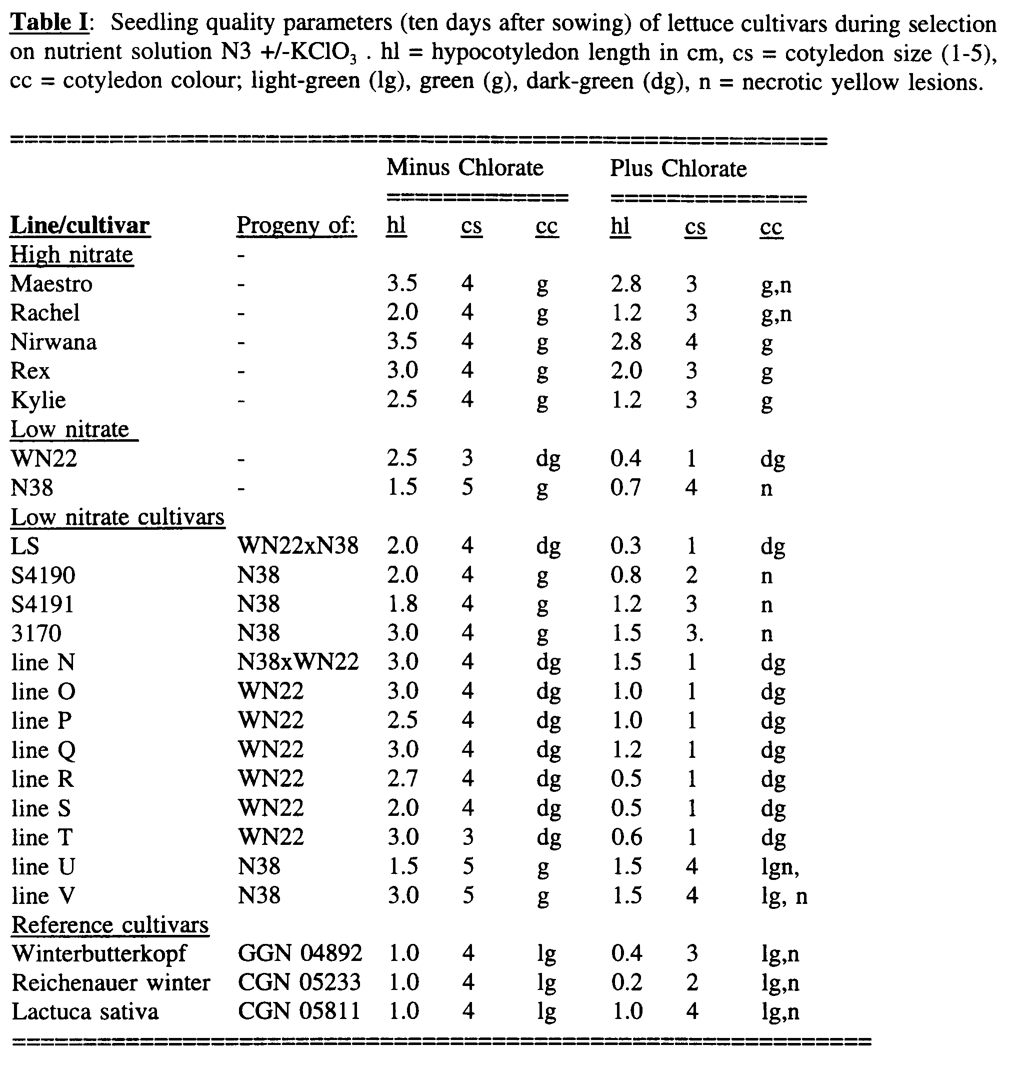

It will be appreciated that the present invention is not limited to the above description or WN22 deposit only.

## Claims

1. Lettuce heads which contain less than or equal to 0.25% (w/w) nitrate at the time of harvest, the plants from which they are obtained having been grown and harvested under natural light at a day-length of not more than 10.5 hours.

2. Lettuce plants, the heads of which contain less than or equal to 0.25% (w/w) nitrate at the time of harvest, the plants having been grown and harvested under natural light at a day-length of not more than 10.5 hours, or lettuce seeds or other propagating material capable of yielding such plants.

3. Lettuce plants the seedlings of which have dark green cotyls when grown in the presence of about 10mM alkaline metal chlorate.

4. Lettuce heads obtained from the plants of the preceding claim, which heads contain less than or equal to 0.25% (w/w) nitrate at the time of harvest, the plants from which they are obtained having been grown and harvested under natural light at a day-length of not more than 10.5 hours.

5. Lettuce roots containing an NADH dependant nitrate reductase which, in the presence of non-limiting quantities of nitrate, is capable of catalyzing the formation of at least 3.7µmol nitrite per gram fresh weight of root per hour.

6. Lettuce heads containing an NADPH dependant nitrate reductase which, in the presence of non-limiting quantities of nitrate, is capable of catalyzing the formation of at least 6.1µmol nitrite per gram fresh weight of head per hour.

7. Lettuce plants comprising the roots of claim 5 and the heads of claim 6.

8. A method of selecting low nitrate lettuces comprising germinating lettuce seeds or growing the resulting seedlings in the presence of about 10mM alkaline metal chlorate and selecting those seedlings which have non necrotized - substantially shrunken cotyledons, and substantially unextended hypocotyls.

9. Low nitrate lettuce plants obtainable according to the preceding claim.

10. Lettuce plants according to either of claims 7 or 9, the heads of which contain less than or equal to 0.25% (w/w) nitrate at the time of harvest, the plants having been grown and harvested under natural light at a day-length of not more than 10.5 hours.

11. Lettuce heads according to any one of claims 1,2,4,6 and 10, which weigh at least 60g at the time of harvest.

12. Lettuce plants according to any one of claims 1 to 4 and 7, 9 and 10, the heads of which contain between 0.20 and 0.25% (w/w) nitrate at the time of harvest, the plants having been grown and harvested under natural light at a day-length of between 7.5 and 10.5 hours.

13. A process for obtaining lettuce plants according to any one of claims 1 to 4, 7, 9, 10 and 12, comprising the steps of:
(i) crossing
(a) line N38 or the progeny of a cross of line N38 with a lettuce plant having the desired agronomical value, any F2 and subsequent generations of said progeny having been selected for low nitrate content;
with
(b) line WN22 or the progeny of a cross of line WN22 with a lettuce plant having the desired agronomical value, any F2 and subsequent generations of said progeny having been selected for low nitrate content;
(ii) harvesting the seeds of said cross, growing them and selecting the lettuce plants with heads having a low nitrate content;
(iii) optionally crossing selected plants from step (ii) with lettuce plants having the desired agronomical value, and selecting from the progeny of the latter cross plants having heads with a low nitrate content;
(iv) selfing the selected plants according to steps (ii) or (iii) repeatedly and selecting in each generation the plants having heads with a low nitrate content;
(v) whereby selfed plants obtained and selected according to step (iv) may be crossed with lettuce plants having the desired agronomical value, provided the progeny thereof is screened for nitrate content, and the plants of the progeny having a low nitrate content, are then selfed and selected according to step (iv) and such selfing process repeated till lettuce plants with heads of the desired nitrate content are obtained.

14. The process of the preceding claim, wherein in each selection step for low nitrate content of a generation the selected plants are those that have the lowest nitrate content of that generation.

15. The process of either of claims 13 or 14, wherein the progeny of any cross with lettuce plants having a desired agronomical value in steps (i), (iii) and/or (v) is selfed to at least the F3 generation and/or wherein the selfing according to step (iv) to the final product is carried out up to at least the F4 generation.

16. A genetic complex comprising the trait for low nitrate content provided by line N38 and the trait for low nitrate content provided by line WN22.

17. A genetic complex according to the preceding claim, wherein the said traits are provided by the nitrate reductase gene loci in each of the lines.

18. A plant comprising the complex of the preceding claim and the use of the said complex in the production of lettuce plants having heads with a nitrate content of less than or equal to 0.25% (w/w) at the time of harvest, the plants having been grown and harvested under natural light at a day-length of not more than 10.5 hours.
